(19) Europäisches Patentamt
European Patent Office
Office européen des brevets

(11) **EP 3 189 454 B1**

(12) **FASCICULE DE BREVET EUROPEEN**

(45) Date de publication et mention
de la délivrance du brevet:
**04.11.2020 Bulletin 2020/45**

(51) Int Cl.:
***G16H 50/50*** *(2018.01)*     ***G16H 20/30*** *(2018.01)*

(21) Numéro de dépôt: **15760125.3**

(22) Date de dépôt: **28.08.2015**

(86) Numéro de dépôt international:
**PCT/EP2015/069800**

(87) Numéro de publication internationale:
**WO 2016/034520 (10.03.2016 Gazette 2016/10)**

(54) **PROCÉDÉ DE SIMULATION DE STIMULATION CÉRÉBRALE, DISPOSITIF ET PROGRAMME D'ORDINATEUR CORRESPONDANT**

VERFAHREN ZUR SIMULATION DER HIRNSTIMULATION, ZUGEHÖRIGES COMPUTERPROGRAMM UND VORRICHTUNG

METHOD FOR SIMULATING BRAIN STIMULATION, CORRESPONDING COMPUTER PROGRAM AND DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.09.2014 FR 1458310**

(43) Date de publication de la demande:
**12.07.2017 Bulletin 2017/28**

(73) Titulaires:
• **Université de Rennes I**
  **35065 Rennes Cedex (FR)**
• **Centre Hospitalier Universitaire Pontchaillou**
  **35000 Rennes (FR)**
• **Institut National de la Santé et de la Recherche Médicale (I.N.S.E.R.M.)**
  **75654 Paris Cedex 13 (FR)**

(72) Inventeurs:
• **JANNIN, Pierre**
  **F-35000 Rennes (FR)**

• **HAEGELEN, Claire**
  **F-35760 Montgermont (FR)**
• **ZHAO, Yulong**
  **F-35000 Rennes (FR)**

(74) Mandataire: **Vidon Brevets & Stratégie**
  **16B, rue de Jouanet**
  **BP 90333**
  **35703 Rennes Cedex 7 (FR)**

(56) Documents cités:
• **FLORENT LALYS ET AL: "Anatomo-clinical atlases correlate clinical data and electrode contact coordinates: Application to subthalamic deep brain stimulation", JOURNAL OF NEUROSCIENCE METHODS, vol. 212, no. 2, 30 janvier 2013 (2013-01-30), pages 297-307, XP055190165, ISSN: 0165-0270, DOI: 10.1016/j.jneumeth.2012.11.002**

**Description**

**1. Domaine de l'invention**

L'invention se rapporte au domaine des dispositifs médicaux. L'invention se rapporte plus particulièrement au domaine des dispositifs médicaux d'analyse et de calcul préparatoire. L'invention se rapporte plus spécifiquement au domaine du calcul préparatoire de traitement, notamment pour l'implantation d'électrodes en zone cérébrale profonde, dans le traitement de la maladie de Parkinson, des dystonies, des Troubles Obsessionnels Compulsifs, de la dépression sévère, du syndrome de Gilles de la Tourette, de certaines addictions, de l'anorexie mentale ou encore de l'hétéro-agressivité.

**2. Art antérieur**

La stimulation cérébrale profonde est une méthode invasive consistant à implanter des électrodes permettant de délivrer, en continu, un courant électrique de faible intensité dans certaines structures spécifiques situées en profondeur du cerveau. Un tel traitement peut être utilisé pour traiter un certain nombre de maladies, tel qu'évoqué précédemment.

La stimulation cérébrale profonde (SCP) est utilisée en tant que technique chirurgicale pour des patients souffrant de troubles résistants aux traitements médicamenteux. Ainsi, ce traitement est majoritairement utilisé lorsque les autres voies de traitement de la maladie se sont révélées être inefficaces. Le défi de la SCP est de trouver l'emplacement optimal pour la stimulation avec la meilleure réduction des symptômes, tout en minimisant les effets secondaires, tels que le déclin cognitif.

Du point de vue de la technique opératoire, la stimulation cérébrale profonde nécessite une implantation d'électrodes. Pour ce faire, une imagerie cérébrale est réalisée afin de visualiser la cible de la stimulation. Des repères sont mis en place sur la peau du crâne pour effectuer un repérage tridimensionnel. Un trajet de l'implantation peut alors être déterminé, en tenant compte de la position des vaisseaux sanguins afin de les éviter.

La procédure d'implantation est mise en œuvre sous anesthésie locale, avec un patient éveillé ayant interrompu des éventuels traitements médicamenteux en cours afin d'évaluer précisément les effets produits par la mise en place des électrodes. Les électrodes sont introduites à travers une ouverture effectuée dans le crâne du patient et sont positionnées dans la cible par exemple à l'aide d'une reconstruction tridimensionnelle du cerveau du patient ou encore sous contrôle direct d'une IRM. Les électrodes sont ensuite fixées au crâne et sont tunnelisées sous la peau jusqu'au lieu d'implantation d'un boîtier (généralement en zone sous-claviculaire). Ce dernier est mis en place sous la peau et connecté aux électrodes.

La problématique liée à la SCP se situe principalement au niveau des résultats obtenus à l'issue de l'implantation. En effet, ces résultats varient d'un patient à l'autre. L'identification anatomique des cibles est difficile même avec les meilleures techniques d'imagerie médicale. La localisation de la cible est donc souvent approximée à partir de connaissances a priori. Par ailleurs des résultats positifs concernant certains aspects de la pathologie (par exemple réduction des tremblements) peuvent être liés à des dégradations (par exemple prise de poids, apathie, hyper activité). Dès lors, pour le praticien, la SCP se résume souvent à une estimation d'une ou plusieurs zones à stimuler, en la constatation des résultats obtenus résultant en une adaptation des paramètres de stimulation (intensité du courant ou changement de plot de l'électrode stimulé). Bien entendu, certaines cibles anatomiques sont connues pour réduire tel ou tel symptôme d'une pathologie, mais les effets secondaires négatifs sont très souvent observés. La stratégie choisie est donc toujours un compromis entre réduction des symptômes de la maladie et minimisation des différents effets secondaires possibles. Dès lors, il existe un besoin d'un outil permettant de prendre en compte les multiples aspects de la pathologie et d'expliciter ce compromis, afin de prendre la meilleure décision au sens des résultats cliniques pour planifier au mieux la stimulation cérébrale profonde. Le document "FLORENT LALYS ET AL: Anatomo-clinical atlases correlate clinical data and electrode contact coordinates: Application to subthalamic deep brain stimulation, JOURNAL OF NEUROSCIENCE METHODS, vol. 212, no. 2, 30 janvier 2013 (2013-01-30), pages 297-307" divulgue une procédé représentant l'état de la technique le plus proche.

**3. Résumé de l'invention**

La technique décrite ne comprend pas ces inconvénients de l'art antérieur. Plus particulièrement, la technique décrite inverse la manière dont la stimulation cérébrale est envisagée. La technique décrite permet d'obtenir une indication de localisation de stimulation en fonction d'un résultat/compromis attendu. La technique se rapporte ainsi à un procédé de simulation d'une stimulation cérébrale, délivrant une estimation d'une zone spatiale cible de stimulation, procédé caractérisé en ce qu'il comprend au moins une itération des étapes suivantes :

- sélection, parmi au moins deux valeurs disponibles, d'une valeur à affecter à un score prédéterminé ;
- identification, au sein d'un atlas anatomo-clinique appartenant à un ensemble d'atlas anatomo-cliniques, d'une zone spatiale susceptible de délivrer une valeur proche de ladite valeur à affecter audit score prédéterminé ;

ladite au moins une itération délivrant un ensemble de zones spatiales ;
le procédé étant en outre caractérisé en ce qu'il comprend :

- un calcul, en fonction dudit ensemble de zones spatiales, d'au moins une zone spatiale cible, susceptible de produire un résultat donné représentatif d'au moins une valeur sélectionnée.

Ainsi, on inverse la méthode traditionnelle d'estimation des résultats d'une stimulation : plutôt que de simuler un résultat en fonction d'une zone spécifique préalablement choisie, on simule une zone en fonction de résultats préalablement sélectionnés. Une telle technique permet d'inclure plus facilement, les attentes des utilisateurs vis-à-vis de la simulation. Les valeurs sélectionnées, qui doivent être affectées aux scores, sont soit des valeurs définies, relativement précises, soit des plages de valeurs.

Selon un mode de réalisation particulier, le calcul de ladite zone spatiale cible comprend une intersection, au sein d'un volume anatomique normé, desdites zones spatiales dudit ensemble de zones spatiales.

Selon une caractéristique particulière, ledit ensemble d'atlas anatomo-cliniques est composé d'atlas anatomo-cliniques fonctionnels du cerveau, associant chacun, à une ou plusieurs zone spatiales données, une ou plusieurs fonctions anatomiques et comprenant, pour ces zones spatiales données une valeur représentative d'une évolution d'une pathologie lorsque ces zones spatiales sont stimulées.

Selon un mode de réalisation particulier, ledit procédé comprend, préalablement à ladite au moins une itération, une étape de construction dudit ensemble d'atlas anatomo-cliniques.

Selon une caractéristique particulière, ladite étape de construction tient compte d'au moins une caractéristique d'un patient pour lequel ledit procédé de simulation est mis en œuvre.

Selon une caractéristique particulière, ladite zone spatiale cible est la zone spatiale représentative de l'intersection avec le nombre le plus élevé de zones spatiales dudit ensemble de zones spatiales.

La technique proposée se rapporte également à un dispositif de simulation d'une stimulation cérébrale, délivrant une estimation d'une zone spatiale cible de stimulation.

Un tel dispositif comprend :

- des moyens de sélection, parmi au moins deux valeurs disponibles, d'une valeur à affecter à un score prédéterminé ;
- des moyens d'identification, au sein d'un atlas anatomo-clinique appartenant à un ensemble d'atlas anatomo-cliniques, d'une zone spatiale susceptible de délivrer une valeur proche de ladite valeur à affecter audit score prédéterminé ;
délivrant un ensemble de zones spatiales ;
- des moyens de calcul, en fonction dudit ensemble de zones spatiales, d'au moins une zone spatiale cible, susceptible de produire un résultat donné représentatif d'au moins une valeur sélectionnée.

Par ailleurs, dans le cas de la psycho-chirurgie, d'autres modalités d'interventions non invasives peuvent également bénéficier de la technique proposée, comme par exemple la radio-chirurgie et les ultrasons focalisés de haute-intensité.

Selon une implémentation préférée, les différentes étapes des procédés selon l'invention sont mises en œuvre par un ou plusieurs logiciels ou programmes d'ordinateur, comprenant des instructions logicielles destinées à être exécutées par un processeur de données d'un module relais selon l'invention et étant conçu pour commander l'exécution des différentes étapes des procédés.

En conséquence, l'invention vise aussi un programme informatique, susceptible d'être exécuté par un ordinateur ou par un processeur de données, ce programme comportant des instructions pour commander l'exécution des étapes d'un procédé tel que mentionné ci-dessus.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

L'invention vise aussi un support d'informations lisible par un processeur de données, et comportant des instructions d'un programme tel que mentionnées ci-dessus.

Le support d'informations peut être n'importe quelle entité ou dispositif capable de stocker le programme. Par exemple, le support peut comporter un moyen de stockage, tel qu'une ROM, par exemple un CD ROM ou une ROM de circuit microélectronique, ou encore un moyen d'enregistrement magnétique, par exemple une disquette (floppy disc), un disque dur, un SSD, *etc.*

D'autre part, le support d'information peut être un support transmissible tel qu'un signal électrique ou optique, qui peut être acheminé via un câble électrique ou optique, par radio ou par d'autres moyens. Le programme selon l'invention peut être en particulier téléchargé sur un réseau de type Internet.

Alternativement, le support d'information peut être un circuit intégré (type ASIC ou FPGA) dans lequel le programme est incorporé, le circuit étant adapté pour exécuter ou pour être utilisé dans l'exécution du procédé en question.

Selon un mode de réalisation, l'invention est mise en œuvre au moyen de composants logiciels et/ou matériels. Dans cette optique, le terme "module" peut correspondre dans ce document aussi bien à un composant logiciel, qu'à un composant matériel ou à un ensemble de composants matériels et logiciels.

Un composant logiciel correspond à un ou plusieurs programmes d'ordinateur, un ou plusieurs sous-programmes d'un programme, ou de manière plus générale à tout élément d'un programme ou d'un logiciel apte à mettre en œuvre une fonction ou un ensemble de fonctions, selon ce qui est décrit ci-dessous pour le module concerné. Un tel composant logiciel est exécuté par un processeur de données d'une entité physique (terminal, serveur, passerelle, routeur, etc.) et est susceptible d'accéder aux ressources matérielles de cette entité physique (mémoires, supports d'enregistrement, bus de communication, cartes électroniques d'entrées/sorties, interfaces utilisateur, etc.).

De la même manière, un composant matériel correspond à tout élément d'un ensemble matériel (ou hardware) apte à mettre en œuvre une fonction ou un ensemble de fonctions, selon ce qui est décrit ci-dessous pour le module concerné. Il peut s'agir d'un composant matériel programmable ou avec processeur intégré pour l'exécution de logiciel, par exemple un circuit intégré, une carte à puce, une carte à mémoire, une carte électronique pour l'exécution d'un micrologiciel (firmware), etc. La présente invention divulgue un dispositif et un procédé tels que définis par les revendications 1 et 7.

### 4. Figures

D'autres caractéristiques et avantages de l'invention apparaîtront à la lecture de la description suivante et des dessins annexés, dans lesquels :

- la figure 1 présente les principales étapes de la technique proposée ;
- La figure 2 représente schématiquement un dispositif pour la mise en œuvre du procédé décrit préalablement.

### 5. Description

5.1. Rappel du principe de l'invention

Le principe général de la technique décrite est double. D'une part il consiste à autoriser l'utilisateur (le praticien par exemple, voire le patient lui-même) à sélectionner un ensemble de valeurs de résultats *souhaités* (c'est-à-dire un ensemble de valeurs associées à des résultats que l'on souhaite obtenir à l'issue de l'implantation de l'électrode). Ces résultats sont insérés dans une structure de données. Ils portent par exemple sur la réduction de tremblement, la facilité d'élocution, etc. Choisir un résultat est en soi un changement de paradigme fondamental. D'autre part, la technique consiste à prendre comme base un résultat attendu pour proposer et calculer une solution interventionnelle. On change donc, avec la technique décrite, la manière de planifier une stimulation. Il est important de préciser que la technique décrite ne change nullement le libre arbitre du praticien qui reste libre de pratiquer une implantation différente de celle calculée. Le praticien est également libre de déterminer, par lui-même une implantation qui est identique à celle obtenue par la mise en œuvre de la technique décrite. La technique décrite étant une technique de simulation, elle n'est nullement essentielle à la mise en œuvre de la stimulation. Elle permet cependant d'envisager celle-ci de manière différente.

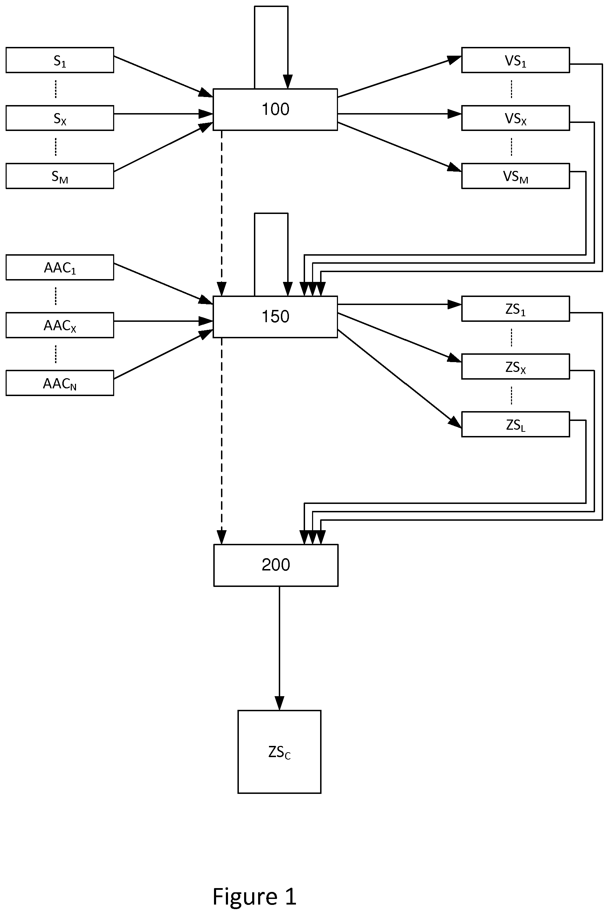

Le principe général de la technique décrite est exposé en relation avec la figure 1. Les résultats souhaités (RS), comme cela est explicité par la suite, sont utilisés pour créer une simulation (10) à partir de scores cliniques ($SC_{[1..M]}$) M représentant le nombre de scores cliniques qui sont représentatifs d'un certain nombre d'interventions et de stimulations passées, lesquelles sont synthétisées (P5) dans des atlas ($AAC_{[1..N]}$) anatomo-cliniques du cerveau, N représentant le nombre d'atlas. Il s'agit d'Atlas fonctionnels qui permettent d'associer une fonction (motrice, cérébrale, biologique, etc.) à un ensemble de données. Un atlas fonctionnel anatomo-clinique du cerveau comprend un ensemble de données. Il s'agit d'une représentation numérique d'un lien entre une anatomie et une fonction. Un tel atlas est utilisable tant pour la stimulation cérébrale profonde que pour la neurostimulation en général (incluant la stimulation magnétique), mais aussi pour la radio chirurgie, et la chirurgie classique.

Ainsi, un préalable à la technique proposée est de disposer de tels atlas anatomo-cliniques. Par exemple, on dispose d'un atlas quantifiant le pourcentage de réduction des tremblements en fonction de la zone de stimulation. Un tel atlas est construit en combinant les données provenant d'un nombre plus ou moins important de stimulations antérieures. Les atlas ont plusieurs typologies (atlas binaires/atlas statistiques, atlas figé/atlas dynamique). Les atlas binaires et les atlas statistiques sont décrits par la suite. Concernant la variabilité de l'atlas, les caractéristiques suivantes peuvent être explicitées : l'atlas est dit « figé » lorsqu'il est précalculé. L'atlas est dit dynamique lorsqu'il est calculé spécifiquement en fonction de certaines caractéristiques du patient (par exemple : âge, sexe, poids, données anatomiques, données génétiques, degrés des symptômes) qui sont fournies préalablement à la simulation. L'avantage de l'atlas dynamique dans le cas de la technique de simulation de l'invention est qu'il s'adapte aux caractéristiques du patient simulé. L'inconvénient est d'une part lié au risque de disposer d'une base de données moins importante pour la création de l'atlas et d'autre part que si le processus de calcul de l'atlas étant long, la simulation peut être également longue.

Quoi qu'il en soit, on dispose, en entrée, d'un ensemble d'Atlas ($AAC_{[1..N]}$) ayant chacun l'attribution d'une valeur d'évolution d'une fonction lié à une zone spatiale stimulée. Cette valeur d'évolution peut être un pourcentage (l'amélioration ou de détérioration) ou une valeur binaire (oui/non, important/pas important, etc.) ou une valeur discrète (-1 : détérioration, 0 : pas de changement, 1 amélioration). Lorsque l'atlas est composé de voxels anatomiques, chaque voxel (ou groupe de voxels), comprend une telle valeur d'évolution de la fonction associée à cet atlas. Ainsi, un composant important pour obtenir les résultats de la technique proposée est l'utilisation d'atlas anatomo-cliniques fonctionnels.

Sur la base de cet ensemble d'atlas de départ, on sélectionne (100), à l'aide d'un ensemble de sélecteurs ($ES_{[1..N]}$), des valeurs souhaitées. Chaque sélecteur ($ES_X$) est associé à un atlas ($AAC_X$) (il est également possible qu'un sélecteur soit associé à plusieurs Atlas et l'inverse qu'un Atlas soit associé à plusieurs sélecteurs, en fonction des scores cliniques auxquels on se réfère). Il y a par exemple un sélecteur « de tremblements » associé à l'atlas relatif aux tremblements, dans lequel, pour des zones spatiales (voxels ou groupes de voxels) un pourcentage d'évolution des tremblements est présent. Dans ce sélecteur « tremblement », on sélectionne une valeur de réduction (ou plus rarement d'augmentation) des tremblements.

Cette étape de sélection est réalisée pour tout ou partie des sélecteurs disponibles afin d'obtenir un ensemble de valeurs de sélection ($VS_{[1..L]}$), L représentant le nombre de valeurs, ce nombre étant inférieur ou égal au nombre de sélecteurs (il est possible de ne sélectionner qu'une partie des valeurs avec certains sélecteurs pour effectuer la simulation).

Sur la base de ces valeurs de sélection ($VS_{[1..L]}$), on calcule (200) une zone spatiale cible ($ZS_C$), qui résulte de l'intersection (250) des zones spatiales ($ZSA_{[1..L]}$) des atlas ($AAC_{[1..L]}$) correspondants aux valeurs sélectionnées. Cette zone spatiale cible ($ZS_C$) représente dans l'idéal, la zone qui correspond aux valeurs sélectionnées dans le sélecteur. Bien entendu, ce cas ne se produit que rarement et il est plus probable que la zone spatiale cible ($ZS_C$) représente l'intersection d'un sous ensemble d'atlas pour lesquels les valeurs sélectionnées dans les sélecteurs sont possibles. Les autres valeurs (celles pour lesquelles une intersection n'a pas été possible) ne sont dans ce cas : soit pas prises en comptes ; soit automatiquement mises à jour pour représenter quelles sont les conséquences de la stimulation de la zone spatiale cible. Par exemple, une zone spatiale cible est trouvée pour la réduction des tremblements et la limitation de la perte d'élocution. Cependant cette zone spatiale cible induit une prise de poids (déterminé par report de la zone spatiale cible dans l'atlas associé à la prise de poids): la valeur du sélecteur associé à la prise de poids peut être automatiquement modifiée par le système pour passer de « absence probable de prise de poids » a « prise de poids très probable ».

En d'autres termes, on inverse la manière dont la stimulation ou la chirurgie est envisagée. En effet, plutôt que de chercher à prédire un résultat d'une stimulation en fonction d'une stratégie d'intervention, la technique proposée utilise un résultat recherché (souhaité) pour calculer une stratégie de stimulation. Cette stratégie de stimulation peut alors éventuellement être mise en œuvre (s'agissant, on le rappelle d'une simulation). La technique proposée permet dès lors d'offrir un libre choix au patient : le patient détermine, éventuellement avec l'aide de son médecin, le résultat qu'il souhaite (et qu'il est possible, selon les connaissances disponibles) atteindre dans le traitement de sa maladie.

En d'autres termes, la méthode proposée comprend, au moins une itération des étapes suivantes :

- une sélection (100), parmi au moins deux valeurs disponibles, d'une valeur ($VS_X$) à affecter à un score ($S_X$) prédéterminé ; cette sélection est effectuée par l'intermédiaire d'un sélecteur qui, pour un score ou un ensemble de scores (correspondant à un résultat attendu), permet de sélectionner l'importance de celui-ci (par exemple amélioration des fonctions motrices antérieures) ; cette valeur ($VS_X$) est en quelque sorte une pondération du score ($S_X$) dans le processus d'intersection postérieur.
- un calcul (200), en fonction de ladite au moins une valeur sélectionnée ($VS_X$), d'au moins une zone spatiale cible ($ZS_C$), au sein d'un volume cérébral donné, susceptible de produire un résultat donné.

Le calcul mis en œuvre se base sur une utilisation inversée et pondérée des atlas anatomo-cliniques du cerveau (préalablement obtenus) pour permettre d'obtenir une cartographie de la zone d'activation idéale pour la stimulation, en fonction des scores cliniques (des résultats) que l'utilisateur souhaite favoriser.

Une composante de l'invention porte sur une méthode de création (P5) de ces atlas fonctionnels anatomo-cliniques du cerveau. Comme cela est détaillé par la suite, ces atlas peuvent se présenter sous au moins deux formes différentes : forme binaire ou forme statistique. L'atlas binaire est un atlas construit avec un critère d'inclusion préalable (résultats considérés comme satisfaisants). Cela signifie que pour un ou plusieurs critères d'inclusion donnés, la valeur associée à un voxel de l'atlas représente la fraction des patients qui remplissent les critères d'inclusion par rapport au nombre total de patients. Il y a ainsi plusieurs cartes (une pour chaque score clinique). L'atlas statistique est un atlas général dans lequel on ne définit pas de critère d'inclusion. Il s'agit d'un atlas généraliste dans lequel la valeur associée à un voxel est représentative des valeurs prises par ce score dans la population de patients analysés.

Dans la suite on décrit un mode de réalisation de la technique dans lequel les atlas sont dédiés à la stimulation cérébrale profonde dans le cas de la maladie de Parkinson. Il est clair, cependant que la technique de simulation proposée peut être mise en œuvre dans d'autres types de pathologie afin de simuler une stimulation en fonction de résultats escomptés a priori.

5.2. Description d'un mode de réalisation

Dans ce mode de réalisation, la technique est matérialisée par une mise en œuvre d'un système d'aide à la décision pour le choix de la cible en neurostimulation (NS). Bien qu'il soit décrit dans le cadre d'une neurostimulation cérébrale interne profonde, un tel système d'aide à la décision peut également être mis en œuvre pour toute technique de stimulation cérébrale interne ou externe, pour peu qu'une base de données d'atlas puisse être utilisée pour effectuer des calculs de stratégies de stimulations en fonction de résultats escomptés (un mode de réalisation de tels atlas est présenté ci-après). L'utilisateur est soit le chirurgien, le neurologue ou le patient lui-même. La décision est prise grâce à un processus utilisant des données prédictives calculées à partir de l'analyse de données cliniques rétrospectives que sont les atlas anatomo cliniques.

Dans ce mode de réalisation de la technique proposée, un sélecteur est utilisé pour faire varier des valeurs de résultats associées à des scores cliniques souhaitées à l'issue de l'intervention. L'utilisateur définit des priorités entre les résultats cliniques attendus. Ces priorités sont traduites en pondérations utilisées pour calculer une stratégie correspondante au respect de ces priorités (avec les pondérations choisies). L'utilisateur peut également fournir des caractéristiques du patient (poids, taille, sexe, âge, antécédents, degré d'atrophie cérébrale, etc.) pouvant être utilisée de manière complémentaire en fonction des atlas à disposition.

Les résultats cliniques attendus, matérialisés dans le sélecteur, peuvent être directement des valeurs quantitatives de scores cliniques mesurables individuellement, ou des catégories regroupant des ensembles cohérents de scores cliniques. De telles catégories peuvent être définies par apprentissage non supervisée (KMeans) : ceci présente un intérêt quand de nombreux scores cliniques sont utilisés et quand ces scores cliniques ne sont pas forcément liés intuitivement. Dès lors un apprentissage par l'intermédiaire d'un circuit d'apprentissage préalable peut être intéressant.

Les priorités (c'est-à-dire les différentes valeurs prioritaires dans les sélecteurs) peuvent être choisies entre des valeurs continues, ou entre des valeurs discrètes, voire des valeurs qualitatives, lorsque celles-ci sont intégrées ou dérivables des atlas utilisés. Les priorités sont définies comme étant les domaines sur lesquels l'utilisateur attend une amélioration clinique par rapport à un état de départ identifié comme la pathologie à soigner.

Bien entendu, les choix des valeurs effectuées dans le sélecteur peuvent contraindre les choix possibles pour les résultats cliniques restant à choisir : par exemple, il est possible qu'une sélection portant sur la réduction des effets secondaires, comme la rapidité de la locution, entraine nécessairement une restriction quant à la réduction des tremblements (cas de la maladie de Parkinson). L'interdépendance des critères de sélection présente dans le sélecteur est partiellement prédéterminée (par l'intermédiaire d'une structure de donnée d'interdépendance gérée par le système, ou directement par calcul sur les atlas ; dans ce cas, les étapes (100) et (200) sont réalisées en mode itératif et interactif). Cette interdépendance, de manière complémentaire, est par ailleurs assujettie aux caractéristiques du patient et par voie de conséquence à la correspondance qui est construite entre des caractéristiques du patient (taille, poids, âge, antécédents, volume cérébral, identification préalable des zones cérébrales dans le volume) et le ou les atlas de la base de données d'atlas correspondant le mieux à la situation de ce patient.

Lorsque les choix de l'utilisateur sont effectués à l'aide du sélecteur, le système met en œuvre un processus de calcul d'une ou plusieurs stratégies d'intervention permettant de répondre aux souhaits exprimés par l'intermédiaire du sélecteur. Il est également possible qu'aucune stratégie d'intervention ne puisse être mise en œuvre. Auquel cas, le système signale cette impossibilité.

Lorsqu'une ou plusieurs stratégies d'intervention peuvent être mises en œuvre, le système signale, pour chacune de ces stratégies, les zones qui doivent être stimulées. De manière complémentaire, chaque stratégie est accompagnée d'un ou plusieurs scores de confiances (Les scores de confiance expriment les erreurs possibles dans la prédiction des résultats cliniques.). De manière complémentaire, le système fournit des résultats cliniques attendus (prédits) pour cette stratégie.

Dans ce mode de réalisation de l'invention, une partie importante du système réside dans le calcul des résultats associés aux sélections effectuées à l'aide du sélecteur. Dans ce mode de réalisation, la méthode de calcul se base sur des données prédictives calculées à partir de l'analyse de données cliniques rétrospectives. Cette analyse consiste, pour un ensemble de patients homogènes (avec des caractéristiques communes) déjà opérés, à corréler les résultats cliniques post opératoires (amélioration ou dégradation, par exemple) avec la stratégie thérapeutique suivie.

Dans le cas de la stimulation cérébrale profonde, la stratégie obtenue à l'issue des calculs consiste en les coordonnées X,Y,Z du ou des plots des électrodes stimulés (calculés dans un repère anatomique générique) ainsi que des paramètres électriques de stimulation. Dans le cas de la stimulation cérébrale profonde, le résultat clinique correspond à la valeur correspondant à la différence avec et sans stimulation d'un score clinique. Pour chaque score ou groupe de scores, la corrélation score-stratégie résulte en un volume 3D, montrant, en chaque voxel, le résultat clinique si le tissu neuronal inclut dans ce voxel est activé.

Dans ce mode de réalisation, un volume 3D, appelé de prédiction (volume 3D qui est un atlas anatomo-clinique), est disponible par score clinique ou groupe de scores. Il peut s'agir d'un atlas binaire ou d'un atlas statistique, tel que décrits postérieurement. En un même voxel, les résultats cliniques des patients de la population incluse (c'est-à-dire pas nécessairement toute la population de départ, en fonction des critères d'inclusion) peuvent être agrégés (moyenne ou autre valeur statistique). Les valeurs en chaque voxel peuvent être normalisées globalement. Il est aussi possible de conserver en chaque voxel l'écart-type du résultat clinique calculé sur les patients dont le voxel correspond à une zone stimulée. Cet écart-type donne une valeur de confiance sur le résultat clinique prédit. Les volumes de prédiction peuvent être validés par validation croisée. C'est à dire qu'un volume est construit sur une population de N sujets et testé sur un sujet $i$ n'appartenant pas à la population utilisée pour la construction. Le test consiste à comparer la valeur du résultat clinique du sujet $i$ stimulé dans un ensemble de voxels Evx avec le résultat clinique prédit par l'atlas dans un ensemble de voxels Evx reprojetés dans l'atlas après recalage non linéaire pour tenir compte des différences anatomiques. Si ce test est répété m fois, il est possible de calculer la valeur de confiance en chaque voxel ou la valeur de confiance globale à l'atlas. Cette valeur peut être fournie à l'utilisateur.

Les priorités (les valeurs des sélecteurs) choisies par l'utilisateur sont converties en poids (pondération). Chaque poids correspond à un score ou à un ensemble de scores. Pour chaque poids, un sous volume du volume de prédiction du ou des score(s) correspondant est sélectionné. Par exemple, si le score est considéré comme prioritaire, on ne sélectionnera que les voxels donnant des résultats cliniques très satisfaisants (par exemple, avec une amélioration significative seulement). Si le score est considéré comme important mais pas prioritaire, on sélectionnera en plus les voxels donnant des résultats cliniques satisfaisants (par exemple, avec une amélioration légère ou possible). Si le score est considéré comme non-important, on sélectionnera en plus les voxels donnant des résultats cliniques quelconques (par exemple, avec une amélioration non démontrée voire même dégradation) ou tous les voxels du volume.

Les notions de satisfaction sur les résultats cliniques peuvent être définies par des catégories associant à une plage de pourcentages d'amélioration un niveau de satisfaction. Ces plages sont définies par un ou des experts, ou pris dans la littérature. Les plages peuvent être différentes en fonction des scores cliniques. La sélection des voxels du sous volume se fait par simple seuillage des valeurs correspondant dans le volume de prédiction (ou atlas anatomo-clinique).

Par la suite, pour l'ensemble des poids, les sous volumes calculés seront agrégés : par exemple, par intersection simple ou par analyse mathématique plus complexe. L'agrégation des volumes peut être réalisée par simple intersection géométrique des sous volumes extraits de chaque atlas anatomo-clinique. L'agrégation des sous-volumes fournit l'ensemble des possibles : c'est à dire les zones qui une fois stimulées donneront les résultats cliniques correspondant aux priorités choisies. En chaque point de ce volume des possibles, les résultats cliniques attendus (prédits) seront extraits des valeurs des volumes de prédiction de chaque score en ce point. Pour chaque résultat clinique, une valeur de confiance sera aussi disponible : écart-type des résultats en ce point calculé sur la population de patients, par exemple, ou valeur de la puissance de prédiction en chaque score calculé par validation croisée.

### 5.3. Génération des Atlas

Comme indiqué préalablement, la technique proposée se base sur la création d'atlas spécifiques. Un tel atlas est une cartographie des points de l'espace, construite à partir d'IRM et/ou de scanners de nombreux patients ayant subi une stimulation cérébrale profonde. Pour ces patients, on extrait un ou plusieurs points du cerveau qui ont été stimulés. On établit un lien entre ces points et des scores cliniques représentatifs d'un degré de réussite et/ou d'échec de l'intervention sur différents aspects moteurs, sensoriels, etc. Il est ainsi possible d'intégrer au processus d'optimisation de trajectoire une prise en compte de données rétrospectives. A l'inverse, comme c'est le cas de la technique décrite préalablement, il est intéressant de prendre comme base de départ un degré d'évolution de l'état clinique pour en déduire une planification. En d'autres termes, plutôt que d'avoir une estimation du résultat en fonction d'une planification réalisée par le praticien, on sélectionne d'abord un résultat attendu pour en déduire une planification.

Ceci passe par l'utilisation d'atlas spécifiquement construits pour ce type d'approches. Comme indiqué préalablement, deux types d'atlas sont envisageables : l'atlas binaire et l'atlas statistique. Par ailleurs, on suppose que l'on créé un atlas par score clinique. On rappelle que le score clinique est une mesure, à l'aide d'un certain nombre de paramètres, d'un état du patient pour en déduire une valeur. On dispose donc d'un score clinique avant intervention et d'un score clinique après intervention. Les atlas de l'invention sont basés sur ces données préalables : score avant stimulation, zone spatiale de stimulation, score après stimulation. La prescription médicamenteuse pré et post stimulation peut également éventuellement être prise en compte, sans que cela revêt un caractère obligatoire.

### 5.3.1. Données cliniques préalables

Préalablement à la génération proprement dite des atlas, il est nécessaire de réaliser une phase de collecte et de normalisation de données cliniques associable à des points ou à des zones des voxels dans les atlas. Dans ce mode de réalisation de l'invention, se rapportant à la maladie de parkinson, Les échelles de mesure UPDRS (Unified Parkinson's disease rating Scale part II, III and IV), Schwab & England et Hoehn & Yahr sont employées comme scores. Une mesure des symptômes non moteurs (i.e. scores neuro psychologiques) sont également utilisés (MDRS test, MATTIS score, test de fluence verbale catégorielle et phonémique, STROOP test, Trail Making Test (TMT), UPDRS I). De plus, les questionnaires de santé des patients (i.e. SF36, PDQ39) sont ajoutés aux calculs, tout comme que le gain de poids du patient, qui est un autre effet secondaire indésirable de de la stimulation cérébrale profonde. Les patients sont testés cliniquement par un neurologue avant l'intervention et après l'intervention (lorsque la stimulation est active). Chaque échelle, questionnaire et test permet d'obtenir un score (S). Par exemple, un score UPDRS, un score Schwab & England, un score Hoehn & Yahr, un score MDRS, un score MATTIS, etc. et ce pour chaque patient (P). On a donc un ensemble de scores pour chaque patient.

Un pourcentage d'amélioration ou de détérioration peut être défini pour chaque score S :

$$S_{\%} = \frac{S_{dopa-off}^{post} - S_{dopa-off}^{pre} + S_{dopa-on}^{post} - S_{dopa-on}^{pre}}{2 \times (S_{max} - S_{min})} \times 100$$

formule dans laquelle :

- $S_{dopa-off}^{post}$ représente la valeur du score après intervention, sans prescription médicamenteuse complémentaire ;
- $S_{dopa-off}^{pre}$ représente la valeur du score avant intervention, sans prescription médicamenteuse complémentaire ;
- $S_{dopa-on}^{post}$ représente la valeur du score après intervention, avec prescription médicamenteuse complémentaire ;
- $S_{dopa-on}^{pre}$ représente la valeur du score avant intervention, avec prescription médicamenteuse complémentaire ;
- $S_{max}$ représente la valeur maximum du score ;
- $S_{min}$ représente la valeur minimum du score.

Une autre formule de calcul d'un pourcentage d'amélioration ou de détérioration peut être définie pour chaque score S (sans prise en compte de prescription médicamenteuse) :

$$S_{\%} = \frac{S_{dopa-off}^{post} - S_{dopa-off}^{pre}}{(S_{max} - S_{min})} \times 100$$

Ainsi, on dispose, pour les patients inclus dans la génération des Atlas, un ensemble de scores qui peuvent être associés (liés) aux données d'imagerie médicales.

### 5.3.2. Atlas Binaire

L'atlas binaire est un atlas construit avec un critère d'inclusion préalable (de score et non de patient : seule l'amélioration du score est prise en compte, pas la dégradation). Cela signifie que pour un ou plusieurs critères d'inclusion donnés, la valeur associée à un voxel de l'atlas représente la fraction des scores des patients qui remplissent les critères d'inclusion par rapport au nombre total de patients.

Le critère d'inclusion n'est pas un critère d'inclusion patient, mais un seuil par rapport au score. Pour construire ce type d'atlas, le praticien met en œuvre des critères d'inclusion en lien avec les différents tests effectués. Ainsi, en utilisant ces conditions d'inclusion, un Atlas est créé en utilisant tous les scores des patients qui remplissent les conditions d'inclusion pour un test donné. Ainsi, seule une partie des patients est utilisée pour créer ce type d'atlas : les patients chez qui le score a été amélioré au-delà d'un seuil donné.

Typiquement, ce type d'atlas est associé à un sélecteur binaire : dans la phase de sélection, une sélection associée à ce type d'atlas est une sélection binaire. Par exemple, la prise de poids, qui est un facteur dépendant du poids d'origine du patient, peut être traduite par une sélection binaire de type oui/non, sans toutefois que l'on se risque à quantifier cette prise de poids de manière forte.

### 5.3.3. Atlas statistiques

L'atlas statistique diffère de l'atlas binaire en ce qu'il ne se base pas sur des critères d'inclusion. La valeur associée à un voxel ne représente pas une probabilité de survenance, mais une efficacité globale ou la liste de toutes les valeurs d'efficacité mesurées sur les patients. Ce score tient compte du nombre de patients, de la zone de stimulation, des réponses cliniques des patients (mesurées par les tests et scores).

On décide donc de créer des Atlas individuels d'abord, puis éventuellement de les agréger avec des poids dédiés pour créer un Atlas agrégé et multi-partitionné finale. L'équation est la suivante :

$$Map(x, y, z) = \frac{1}{N} \sum_{i=1}^{N} \left( \sum_{k=1}^{M} S_k^i w_k \right) \frac{\gamma_i(x_i, y_i, z_i)}{\sum_{i=1}^{N} \gamma_i(x_i, y_i, z_i)}$$

dans laquelle :

- $N$ représente le nombre de patients ;
- $(x_i, y_i, z_i)$ représente les coordonnées tridimensionnelles stimulées pour le patient $i$ ;
- $M$ représente le nombre de scores cliniques utilisés ;
- $S_k^i$ représente la valeur du score clinique $k$ pour le patient $i$ ;
- $\gamma_i$ représente l'influence tridimensionnelle de la stimulation, pour les coordonnées $(x_i, y_i, z_i)$ ; et
- $w_k$ représente le poids du score clinique $k$.

### 5.4. Calcul de stratégie en fonction des choix effectués dans le sélecteur

Comme indiqué, on souhaite identifier une ou plusieurs zones ou régions anatomiques susceptibles de produire un ou plusieurs résultats attendus par le patient ou le praticien. Il est possible, pour ce faire de procéder selon au moins deux méthodes différentes. Une troisième méthode est également explicitée dans laquelle un calcul de position préalable est réalisé. On rappelle que l'on cherche à détecter, au sein d'une zone du volume cérébral, une zone de stimulation qui se présente sous la forme de coordonnées spatiales de type *(x,y,z)* où la stimulation doit être réalisée. On rappelle également, que cette zone est associée à une valeur de modification d'un score ou de plusieurs scores.

### 5.4.1. Calcul en série (ou itératif)

Dans ce mode de réalisation, on réalise une recherche de position de manière séquentielle. Le principe général est de rechercher, de manière séquentielle, dans chaque base de données associée à chaque score, les coordonnées spatiales qui répondent aux souhaits formulés dans le sélecteur pour ce score. Ainsi, la méthode consiste :

- pour un score clinique courant (S1), rechercher dans l'atlas correspondant à ce score clinique ($A_{S1}$), une zone spatiale ($Z_{S1}$) correspondant à la valeur sélectionnée ($V_{S1}$) dans le sélecteur qui est associé à ce score ($S_{S1}$). Cette zone spatiale ($Z_{S1}$) comprend une ou plusieurs coordonnées spatiales (généralement plusieurs, se présentant sous la forme d'un groupe de coordonnées spatiales).
- pour un score clinique suivant (S2), reporter la zone spatiale ($Z_{S1}$) du score clinique courant (S1), dans l'atlas correspondant à ce score clinique suivant ($A_{S2}$) et obtenir une valeur ou une plage de valeurs ($V_{S2}{}^{[ZS1]}$) associée à ce score clinique suivant (S2) pour cette zone spatiale ($Z_{S1}$) ;
- Puis deux modes sont possibles :

  - A- comparer la valeur ($V_{S2}{}^{[ZS1]}$) associée à ce score clinique suivant (S2) avec une valeur sélectionnée ($V_{S2}$) dans le sélecteur qui est associé à ce score ($S_{S2}$).

    - lorsque la valeur la valeur $V_{S2}{}^{[ZS1]}$ se situe dans une fourchette d'acceptabilité au regard de la valeur sélectionnée ($V_{S2}$), poursuivre le processus en passant au score clinique suivant (S3) ;
    - lorsque la valeur la valeur $V_{S2}{}^{[ZS1]}$ se ne situe pas dans une fourchette d'acceptabilité au regard de la valeur sélectionnée ($V_{S2}$), interrompre le processus ;

- B- modifier les valeurs de saisie possibles du sélecteur qui est associé à ce score ($S_{S2}$) en restreignant les choix possibles pour l'utilisateur à ce qui n'est possible seulement. Puis revenir à la sélection de ($V_{S2}^{[ZS1]}$) par l'utilisateur ...

Le procédé décrit précédemment est récursif. Il se poursuit pour chaque score successif de la liste de score. La condition, bien entendu étant qu'un atlas soit disponible pour ce score. Dans ce mode de réalisation, tant qu'une valeur sélectionnée dans un score de départ est associée à une zone « compatible » avec une zone d'un autre score, le processus se poursuit, jusqu'à identifier tous les scores qui sont associés à cette zone. La technique décrite précédemment est mise en œuvre pour chaque zone spatiale identifiée. Par exemple, il est possible que le premier score pris en compte permette d'identifier plusieurs zones susceptibles de répondre à la requête de l'utilisateur. Dans un tel cas, le procédé est mis en œuvre pour chacune de ces zones.

5.4.2. Calcul en parallèle

Dans ce mode de réalisation, on réalise une recherche de position en parallèle. Le principe général est de rechercher, parallèlement, dans chaque base de données associée à chaque score, les coordonnées spatiales qui répondent aux souhaits formulés dans le sélecteur pour ce score. Ainsi, la méthode consiste en la mise en œuvre d'une pluralité des étapes suivantes :

- pour un score clinique courant (SC), rechercher dans l'atlas correspondant à ce score clinique ($A_{SC}$), une zone spatiale ($Z_{SC}$) correspondant à la valeur sélectionnée ($V_{Sc}$) dans le sélecteur qui est associé à ce score ($S_{SC}$). Cette zone spatiale ($Z_{Sc}$) comprend une ou plusieurs coordonnées spatiales (généralement plusieurs, se présentant sous la forme d'un groupe de coordonnées spatiales).

Lorsque l'ensemble des recherches a été effectué, on obtient une pluralité de zones spatiales ($Z_{Sc}^{[1..n]}$), où n représente le nombre de scores. L'étape suivante consiste à effectuer une intersection de ces zones spatiales ($Z_{Sc}^{[0..n]}$) par rapport à un référentiel commun. Le résultat de cette intersection produit, dans le meilleur des cas, une zone spatiale cible ($Z_{ST}$). Cette zone spatiale cible ($Z_{ST}$) constitue la cible anatomique à viser pour obtenir les résultats escomptés (par l'utilisateur lors de la sélection des résultats avec la pluralité de sélecteurs). Dans le cas le plus défavorable, aucune intersection ne peut être identifiée : cela signifie qu'il n'y a pas de zone spatiale commune aux choix effectués dans les sélecteurs.

Dans une pluralité de cas intermédiaires, seuls certains choix, matérialisés par certaines valeurs de scores, présentent des zones spatiales qui sont plus ou moins identiques. On obtient ainsi une zone spatiale cible partielle ($Z_{STP}$) dans laquelle seule un sous ensemble de scores est pris en compte. Ainsi, une stimulation de zone spatiale cible partielle ($Z_{STP}$) peut entrainer une modification des scores exclus du sous-ensemble de scores pris en compte.

Dans ces cas, les plus nombreux, un calcul « inverse » peut être effectué pour attribuer une nouvelle valeur aux scores dont les zones spatiales ne font pas partie de la zone spatiale cible partielle ($Z_{STP}$).

5.4.3. Calcul automatique

De manière complémentaire, préalablement à l'étape de sélection des valeurs de scores, il est envisagé de réaliser un calcul automatique dont l'objectif est de pré-positionner au moins certaines des valeurs de scores dans les sélecteurs. Ce calcul automatique préliminaire vise à recherche une solution maximisant des valeurs de scores. Ce calcul automatique est effectué sur la base d'une liste ordonnée de score. Cette liste ordonnée de score affecte implicitement une importance aux scores, les uns par rapport aux autres. En fonction des pathologies, en effet, certains scores cliniques peuvent être plus représentatifs que d'autres. Dès lors, ces scores peuvent être ordonnés par importance. Par exemple, dans le cas de la maladie de parkinson, le score relatif aux tremblements peut être considéré comme prépondérant par rapport à celui relatif à la prise de poids.

Ainsi, dans cette variante, une phase préalable de recherche de solution de maximisation des scores est mises en œuvre en effectuant des intersections de zones spatiales ($Z_{Si}$) correspondant des valeurs maximisées ($V_{Si}$) pour ces scores, en partant des scores les plus significatifs de la pathologie aux scores les moins significatifs de la pathologie.

Ces intersections permettent d'obtenir une zone spatiale cible automatique ($Z_{STA}$) à laquelle correspond des valeurs des scores utilisés.

5.5. Dispositif de mise en œuvre

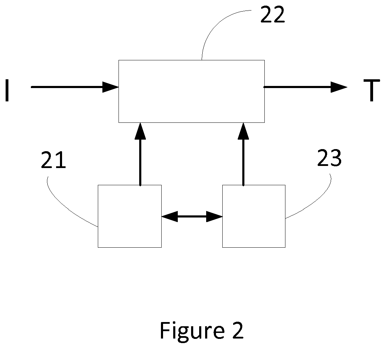

On décrit, en relation avec la figure 2, un dispositif mis en œuvre pour obtenir une simulation de stimulation cérébrale, selon le procédé décrit préalablement. Par exemple, le dispositif comprend une mémoire 21 constituée d'une mémoire tampon, une unité de traitement 22, équipée par exemple d'un microprocesseur, et pilotée par le programme d'ordinateur 23, mettant en œuvre un procédé d'obtention.

À l'initialisation, les instructions de code du programme d'ordinateur 23 sont par exemple chargées dans une mémoire avant d'être exécutées par le processeur de l'unité de traitement 22. L'unité de traitement 22 reçoit en entrée une valeur ($VS_X$) à affecter à un score ($S_X$) prédéterminé et des atlas anatomo-cliniques liés à ces scores. Le microprocesseur de l'unité de traitement 22 met en œuvre les étapes du procédé selon les instructions du programme d'ordinateur 23 pour générer une zone spatiale cible ($ZS_C$), susceptible de produire un résultat donné représentatif d'au moins une valeur sélectionnée ($VS_X$).

Pour cela, le dispositif comprend, outre la mémoire tampon 21, des moyens de communications, tels que des modules de communication réseau, des moyens de transmission de donnée et éventuellement un processeur de chiffrement.

Ces moyens peuvent se présenter sous la forme d'un processeur particulier implémenté au sein du dispositif, ledit processeur étant un processeur sécurisé. Selon un mode de réalisation particulier, ce dispositif met en œuvre une application particulière qui est en charge des calculs.

Ces moyens se présentent également comme des interfaces de communications permettant d'échanger des données sur des réseaux de communication, des moyens d'interrogations et de mise à jour de base de données,...

Plus particulièrement, un tel dispositif comprend :

- des moyens de sélection, parmi au moins deux valeurs disponibles, d'une valeur ($VS_X$) à affecter à un score ($S_X$) prédéterminé ; Les moyens de sélection peuvent se présenter sous la forme de sélecteurs, par exemple des sélecteurs matériels disposés au sein d'une platine, recevant des sélecteurs (sélecteur coulissant continu, sélecteur orientable à plusieurs positions, variateur) ou bien des modules de sélection programmables, se présentant par exemple sous la forme de modules logiciels et/ou matériels, aptes à communiquer, par l'intermédiaire d'un processeur, avec une interface homme/machine pour afficher, sur un moyen de représentation d'information, des sélecteurs virtuels pouvant être manipulé à l'aide d'un dispositiof de saisie ;
- des moyens d'identification, au sein d'un atlas anatomo-clinique ($AAC_X$) appartenant à un ensemble d'atlas anatomo-cliniques ($AAC_{[1..N]}$), d'une zone spatiale ($ZS_X$) susceptible de délivrer une valeur proche de ladite valeur ($VS_X$) à affecter audit score ($S_X$) prédéterminé ; délivrant un ensemble de zones spatiales ($ZS_{[1..L]}$) ; Ces moyens peuvent se présenter sous la formes de modules logiciels et/ou de modules matériels spécifiquement créer pour rechercher, au sein d'un atlas donné, une ou plusieurs zones correspondant à des valeurs transmises par l'intermédiaire des moyens de sélection ;
- des moyens de calcul, en fonction dudit ensemble de zones spatiales ($ZS_{[1..L]}$), d'au moins une zone spatiale cible ($ZS_C$), susceptible de produire un résultat donné représentatif d'au moins une valeur sélectionnée ($VS_X$). De tels moyens se présentent par sous la forme d'un processeur de traitement de données multidimensionnelles, comme par exemple un processeur de type processeur graphique auquel un algorithme de calcul particulier est fourni. Un tel agencement présente l'avantage d'accélérer fortement le traitement. Une autre possibilité peut être d'utiliser une processeur standard ou un microprocesseur spécifiquement réalisé pour effectuer ce type de calcul.

**Revendications**

1. Procédé de simulation d'une stimulation cérébrale mis en oeuvre sur ordinateur, délivrant une estimation d'une zone spatiale cible ($ZS_C$) de stimulation, procédé **caractérisé en ce qu'**il comprend au moins une itération des étapes suivantes :

   - sélection (100), à l'aide d'un sélecteur (SSC), parmi au moins deux valeurs disponibles, d'une valeur ($VS_X$) à affecter à un score ($S_X$) prédéterminé, correspondant à un résultat attendu, ledit score ($S_X$) prédéterminé étant issu d'au moins une donnée clinique préalable mesurée sur au moins un patient ;
   - identification (150), au sein d'un atlas anatomo-clinique ($AAC_X$) appartenant à un ensemble d'atlas anatomo-cliniques ($AAC_{[1..N]}$), d'une zone spatiale ($ZS_X$) susceptible de délivrer une valeur proche de ladite valeur ($VS_X$) à affecter audit score ($S_X$) prédéterminé, ladite identification comprenant au moins une recherche dans l'atlas ($AAC_X$) correspondant à ce score ($S_X$), d'une zone spatiale ($ZS_X$) correspondant à la valeur sélectionnée ($VS_X$) dans le sélecteur (SSC) qui est associé à ce score ; ladite au moins une itération délivrant un ensemble de zones spatiales ($ZS_{[1..L]}$) ;

   le procédé étant en outre **caractérisé en ce qu'**il comprend :

   - un calcul (200), en fonction dudit ensemble de zones spatiales ($ZS_{[1..L]}$), d'au moins une zone spatiale cible (ZS$_C$), susceptible de produire un résultat donné représentatif d'au moins une valeur sélectionnée (VS$_X$), ledit calcul étant mis en œuvre de manière itérative ou en parallèle, ladite au moins une zone spatiale cible (ZS$_C$) résultant d'une intersection d'au moins deux zones spatiales dudit ensemble de zones spatiales (ZS$_{[1..L]}$).

2. Procédé selon la revendication 1, **caractérisé en ce que** ledit ensemble d'atlas anatomo-cliniques (AAC$_{[1..N]}$) est composé d'atlas anatomo-cliniques (AAC$_X$) fonctionnels du cerveau, associant chacun, à une ou plusieurs zone spatiales données, une ou plusieurs fonctions anatomiques et comprenant, pour ces zones spatiales données une valeur représentative d'une évolution d'une pathologie lorsque ces zones spatiales sont stimulées.

3. Procédé selon la revendication 1, **caractérisé en ce qu'**il comprend, préalablement à ladite au moins une itération, une étape de construction (P5) dudit ensemble d'atlas anatomo-cliniques (AAC$_{[1..N]}$).

4. Procédé selon la revendication 3, **caractérisé en ce que** ladite étape de construction (P5) tient compte d'au moins une caractéristique d'un patient pour lequel ledit procédé de simulation est mis en œuvre.

5. Procédé selon la revendication 1, **caractérisé en ce que** ladite zone spatiale cible (ZS$_C$) est la zone spatiale représentative de l'intersection avec le nombre le plus élevé de zones spatiales dudit ensemble de zones spatiales (ZS$_{[1..L]}$).

6. Dispositif de simulation d'une stimulation cérébrale, délivrant une estimation d'une zone spatiale cible (ZS$_C$) de stimulation, dispositif **caractérisé en ce qu'**il comprend :

   - des moyens de sélection (SCC), parmi au moins deux valeurs disponibles, d'une valeur (VS$_X$) à affecter à un score (S$_X$) prédéterminé lequel correspondant à un résultat attendu, , ledit score (S$_X$) prédéterminé étant issu d'au moins une donnée clinique préalable mesurée sur au moins un patient ;
   - des moyens d'identification, au sein d'un atlas anatomo-clinique (AAC$_X$) appartenant à un ensemble d'atlas anatomo-cliniques (AAC$_{[1..N]}$), d'une zone spatiale (ZS$_X$) susceptible de délivrer une valeur proche de ladite valeur (VS$_X$) à affecter audit score (S$_X$) prédéterminé, ladite identification comprenant au moins une recherche dans l'atlas (AAC$_X$) correspondant à ce score (S$_X$), d'une zone spatiale (ZS$_X$) correspondant à la valeur sélectionnée (VS$_X$) dans le sélecteur (SSC) qui est associé à ce score ;

   délivrant un ensemble de zones spatiales (ZS$_{[1..L]}$) ;

   - des moyens de calcul, en fonction dudit ensemble de zones spatiales (ZS$_{[1..L]}$), d'au moins une zone spatiale cible (ZS$_C$), susceptible de produire un résultat donné représentatif d'au moins une valeur sélectionnée (VS$_X$), ledit calcul étant mis en œuvre de manière itérative ou en parallèle, ladite au moins une zone spatiale cible (ZS$_C$) résultant d'une intersection d'au moins deux zones spatiales dudit ensemble de zones spatiales (ZS$_{[1..L]}$).

7. Produit programme d'ordinateur téléchargeable depuis un réseau de communication et/ou stocké sur un support lisible par ordinateur et/ou exécutable par un microprocesseur, **caractérisé en ce qu'**il comprend des instructions de code de programme pour l'exécution d'un procédé de simulation selon la revendication 1, lorsqu'il est exécuté par un processeur.

**Patentansprüche**

1. Simulationsverfahren einer Hirnstimulation, das auf einem Computer umgesetzt wird, das eine Schätzung einer räumlichen Stimulationszielzone (ZS$_c$) liefert, Verfahren **dadurch gekennzeichnet, dass** es mindestens eine Iteration der folgenden Schritte umfasst:

   - Auswahl (100) mit Hilfe eines Auswählers (SSC) aus mindestens zwei verfügbaren Werten eines Werts (VS$_X$), der einem vorbestimmten Score (S$_X$) zuzuweisen ist, der mindestens einem erwarteten Resultat entspricht, wobei der vorbestimmte Score (S$_X$) aus mindestens einem klinischen Datum hervorgeht, das vorab auf mindestens einem Patienten gemessen wird;
   - Identifikation (150) innerhalb eines anatomisch-klinischen Atlas (AAC$_X$), der zu einer Einheit anatomisch-klinischer Atlanten (AAC$_{[1..N]}$) gehört, einer räumlichen Zone (ZS$_X$), die einen Wert nahe dem Wert (VS$_X$), der dem vorbestimmten Score (S$_X$) zuzuweisen ist, liefern kann, wobei die Identifikation mindestens eine Suche in dem Atlas (AAC$_X$), der diesem Score (S$_X$) entspricht, einer räumlichen Zone (ZS$_X$) umfasst, die dem Wert (VS$_X$),

der in dem Auswähler (SSC), der mit diesem Score assoziiert ist, ausgewählt wird;

wobei die mindestens eine Iteration eine Einheit räumlicher Zonen ($ZS_{[1..L]}$) liefert; wobei das Verfahren außerdem **dadurch gekennzeichnet ist, dass** es umfasst:

- eine Berechnung (200) in Abhängigkeit von der Einheit räumlicher Zonen ($ZS_{[1..L]}$) mindestens einer räumlichen Zielzone ($ZS_c$), die ein gegebenes Resultat ergeben kann, das für mindestens einen ausgewählten Wert ($VS_X$) repräsentativ ist, wobei die Berechnung iterativ oder parallel umgesetzt wird, wobei die mindestens eine räumliche Zielzone ($ZS_c$) aus einem Schnittpunkt von mindestens zwei räumlichen Zonen der Einheit räumlicher Zonen ($ZS_{[1..L]}$) resultiert.

2. Verfahren nach Anspruch 1, **dadurch gekennzeichnet, dass** die Einheit anatomisch-klinischer Atlanten ($AAC_{[1..N]}$) aus funktionalen anatomisch-klinischen Atlanten ($AAC_X$) des Hirns besteht, die jeweils mit einer oder mehreren gegebenen räumlichen Zonen eine oder mehrere anatomische Funktionen assoziieren und für diese gegebenen räumlichen Zonen einen Wert umfassen, der für eine Entwicklung einer Pathologie repräsentativ ist, während diese räumlichen Zonen stimuliert werden.

3. Verfahren nach Anspruch 1, **dadurch gekennzeichnet, dass** es vor der mindestens einen Iteration einen Konstruktionsschritt (P5) der Einheit anatomisch-klinischer Atlanten ($AAC_{[1..N]}$) umfasst.

4. Verfahren nach Anspruch 3, **dadurch gekennzeichnet, dass** der Konstruktionsschritt (P5) mindestens ein Merkmal eines Patienten berücksichtigt, für das das Simulationsverfahren umgesetzt wird.

5. Verfahren nach Anspruch 1, **dadurch gekennzeichnet, dass** die räumliche Zielzone ($ZS_c$) die räumliche Zone ist, die für den Schnittpunkt mit der höchsten Anzahl räumlicher Zonen der Einheit räumlicher Zonen ($ZS_{[1..L]}$) repräsentativ ist.

6. Simulationsvorrichtung einer Hirnstimulation, die eine Schätzung einer räumlichen Stimulationszielzone ($ZS_c$) liefert, Vorrichtung **dadurch gekennzeichnet, dass** sie umfasst:

- Mittel (SCC) zur Auswahl aus mindestens zwei verfügbaren Werten eines Werts ($VS_X$), der einem vorbestimmten Score ($S_X$) zuzuweisen ist, der einem erwarteten Resultat entspricht, wobei der vorbestimmte Score ($S_X$) aus mindestens einem klinischen Datum hervorgeht, das vorab auf mindestens einem Patienten gemessen wird;
- Mittel zur Identifikation innerhalb eines anatomisch-klinischen Atlas ($AAC_X$), der zu einer Einheit anatomisch-klinischer Atlanten ($AAC_{[1..N]}$) gehört, einer räumlichen Zone ($ZS_X$), die einen Wert nahe dem Wert ($VS_X$), der dem vorbestimmten Score ($S_X$) zuzuweisen ist, liefern kann, wobei die Identifikation mindestens eine Suche in dem Atlas ($AAC_X$), der diesem Score ($S_X$) entspricht, einer räumlichen Zone ($ZS_X$) umfasst, die dem Wert ($VS_X$) entspricht, der in dem Auswähler (SSC), der mit diesem Score assoziiert ist, ausgewählt ist;
Liefern einer Einheit räumlicher Zonen ($ZS_{[1..L]}$);
- Mittel zum Berechnen in Abhängigkeit von der Einheit räumlicher Zonen ($ZS_{[1..L]}$) mindestens einer räumlichen Zielzone ($ZS_c$), die ein gegebenes Resultat, das für mindestens einen ausgewählten Wert ($VS_X$) repräsentativ ist, ergeben kann, wobei die Berechnung iterativ oder parallel umgesetzt wird, wobei die mindestens eine räumliche Zielzone ($ZS_c$) aus einem Schnittpunkt mindestens zweier räumlicher Zonen der Einheit räumlicher Zonen ($ZS_{[1..L]}$) resultiert.

7. Computerprogrammprodukt, das aus einem Kommunikationsnetz herunterladbar ist und/oder auf einem Träger gespeichert ist, der von einem Computer lesbar ist und/oder von einem Mikroprozessor ausführbar ist, **dadurch gekennzeichnet, dass** es Programmcodeanweisungen für die Ausführung eines Simulationsverfahrens nach Anspruch 1 umfasst, wenn es von einem Prozessor ausgeführt wird.

**Claims**

1. Method for simulating a brain stimulation implemented on a computer, providing an estimate of a target spatial stimulation zone ($ZS_c$), which method is **characterised in that** it comprises at least one iteration of the following steps:

- selecting (100), using a selector (SSC), from at least two available values, a value ($VS_x$) to be allocated to a predetermined score ($S_x$), corresponding to an anticipated result, the predetermined score ($S_x$) originating from

at least one previous clinical data item measured on at least one patient;
- identifying (150), from an anatomical-clinical atlas ($AAC_x$) belonging to an anatomical-clinical atlas set ($AAC_{[1...N]}$), a spatial zone ($ZS_x$) which is capable of providing a value close to the value ($VS_x$) to be allocated to the predetermined score ($S_x$), the identification comprising at least one search in the atlas ($AAC_x$), corresponding to this score ($S_x$), for a spatial zone ($ZS_x$) corresponding to the value ($VS_x$) selected in the selector (SSC) which is associated with this score;

the at least one iteration providing a set of spatial zones ($ZS_{[1..L]}$);
the method being further **characterised in that** it comprises:

- a calculation (200) in accordance with the set of spatial zones ($ZS_{[1..L]}$) of at least one target spatial zone ($ZS_c$) which is capable of producing a specific result representing at least one selected value ($VS_x$), the calculation being carried out in an iterative or parallel manner, the at least one target spatial zone ($ZS_c$) resulting from an intersection of at least two spatial zones of the set of spatial zones ($ZS_{[1..L]}$).

2. Method according to claim 1, **characterised in that** the anatomical-clinical atlas set ($AAC_{[1...N]}$) is composed of functional anatomical-clinical atlases ($AAC_x$) of the brain which each associate, with one or more specific spatial zones, one or more anatomical functions and which comprise, for these specific spatial zones, a value which represents a development of a pathology when these spatial zones are stimulated.

3. Method according to claim 1, **characterised in that** it comprises, prior to the at least one iteration, a step (P5) of constructing the anatomical-clinical atlas set ($AAC_{[1...N]}$).

4. Method according to claim 3, **characterised in that** the construction step (P5) takes into account at least one characteristic of a patient for which the simulation method is implemented.

5. Method according to claim 1, **characterised in that** the target spatial zone ($ZS_c$) is the spatial zone which represents the intersection with the highest number of spatial zones of the set of spatial zones ($ZS_{[1..L]}$).

6. Device for simulating a brain stimulation, providing an estimate of a target spatial stimulation zone ($ZS_c$), which device is **characterised in that** it comprises:

- means (SSC) for selecting, from at least two available values, a value ($VS_x$) to be allocated to a predetermined score ($S_x$) which corresponds to an anticipated result, the predetermined score ($S_x$) originating from at least one previous clinical data item measured on at least one patient;
- means for identifying from an anatomical-clinical atlas ($AAC_x$) belonging to an anatomical-clinical atlas set ($AAC_{[1...N]}$), a spatial zone ($ZS_x$) which is capable of providing a value close to the value ($VS_x$) to be allocated to the predetermined score ($S_x$), the identification comprising at least one search in the atlas ($AAC_x$), corresponding to this score ($S_x$), for a spatial zone ($ZS_x$) corresponding to the value ($VS_x$) selected in the selector (SSC) which is associated with this score;
providing a set of spatial zones ($ZS_{[1..L]}$);
- means for calculating in accordance with the set of spatial zones ($ZS_{[1..L]}$) at least one target spatial zone ($ZS_c$) which is capable of producing a specific result representing at least one selected value ($VS_x$), the calculation being carried out in an iterative or parallel manner, the at least one target spatial zone ($ZS_c$) resulting from an intersection of at least two spatial zones of the set of spatial zones ($ZS_{[1..L]}$).

7. Computer program product which can be downloaded from a communication network and/or stored on a computer readable medium and/or which can be carried out by a microprocessor, **characterised in that** it comprises program encoding instructions for carrying out a simulation method according to claim 1 when it is carried out by a processor.

Figure 1

Figure 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **FLORENT LALYS et al.** Anatomo-clinical atlases correlate clinical data and electrode contact coordinates: Application to subthalamic deep brain stimulation. *JOURNAL OF NEUROSCIENCE METHODS,* 30 Janvier 2013, vol. 212 (2), 297-307